(19) Europäisches Patentamt
European Patent Office
Office européen des brevets

(11) Veröffentlichungsnummer: **0 031 473**
**B1**

(12) **EUROPÄISCHE PATENTSCHRIFT**

(45) Veröffentlichungstag der Patentschrift: **30.05.84**

(51) Int. Cl.³: **C 07 D 235/32**

(21) Anmeldenummer: **80107505.2**

(22) Anmeldetag: **02.12.80**

(54) **Verfahren zur Herstellung von 2-((Alkoxycarbonyl)-amino)-5-(alkylthio)-1H-benzimidazolen.**

(30) Priorität: **04.12.79 HU CI001993**

(43) Veröffentlichungstag der Anmeldung: **08.07.81 Patentblatt 81/27**

(45) Bekanntmachung des Hinweises auf die Patenterteilung: **30.05.84 Patentblatt 84/22**

(84) Benannte Vertragsstaaten: **AT CH DE FR GB IT LI NL**

(56) Entgegenhaltungen:
**DE - A - 2 441 202**
**DE - A - 2 820 375**
**US - A - 4 080 461**

**Organic Chemistry of Sulfur, 1977, Plenum Press, N.Y. p. 124**

(73) Patentinhaber: **CHINOIN Gyogyszer és Vegyészeti Termékek Gyára RT., To utca 1-5, H-1045 Budapest V (HU)**

(72) Erfinder: **Szöke, Sándor, Dipl. Ing. Chem., Vadgesztenye u. 4/C, H-1046 Budapest (HU)**
Erfinder: **Lugosi, György, Dipl. Ing. Chem., Jòzsef u. 9, H-2132 Felsö-Göd (HU)**
Erfinder: **Bakonyi, Mária, Dipl.-Ing. Chem., Munkácsy M. u. 58/a, H-1046 Budapest (HU)**
Erfinder: **Ghyczy, Jenö, Dipl.-Ing. Chem., Michály u.2, H-1016 Budapest (HU)**
Erfinder: **Csermely, György, Dipl.-Ing. Chem., Dongò u.8, H-1149 Budapest (HU)**

(74) Vertreter: **Lotterhos, Hans Walter, Dr.-Ing., Lichtensteinstrasse 3, D-6000 Frankfurt am Main 1 (DE)**

**EP 0 031 473 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung der biologisch aktiven 2-[(Alkoxycarbonyl)-amino]-1H-benzimidazole der allgemeinen Formel V

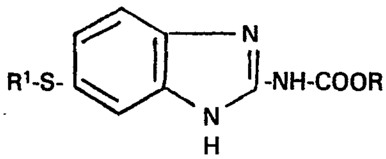

(V)

worin

R $C_{1-3}$-Alkyl und

$R^1$ $C_1$-$C_3$-Alkyl oder Wasserstoff bedeuten.

Die Benzimidazolderivate der allgemeinen Formel V stellen wertvolle Anthelmintica mit breitem Wirkungsspektrum dar. Zu ihrer Herstellung wurde gewöhnlich 4-Alkylthio-phenylen-diamin entweder mit Carbalkoxycyanamid umgesetzt, wobei unmittelbar das Benzimidazolderivat der Formel V erhalten wurde, oder mit Bromcyan zum 2-Amino-5-alkylthio-benzimidazol umgesetzt, das dann mit Alkylchlorformiat in das Benzimidazolderivat der Formel V übergeführt wurde.

Diese Verfahren sind in der US-PS 3 956 499 und der HU-PS 172 538 beschrieben, worin die Herstellung eines der wichtigsten Vertreter dieser Verbindungsgruppe: des 2-[(Methoxycarbonyl)-amin]-5-(propylthio)-1H-benzimidazols näher erläutert ist. Diese Verbindung zeichnet sich durch eine besonders hohe anthelmintische Wirkung mit breitem Wirkungsspektrum aus.

Ein wesentlicher Nachteil dieser vorbekannten Verfahren besteht darin, dass die Herstellung des 4-Alkylthio-1,2-phenylendiamin sehr schwierig und kostspielig ist, toxische und übelriechende Materialien erfordert und gefährliche und teure katalytische Hydrierungsverfahren notwendig macht.

Die Erfindung betrifft ein Verfahren zur Herstellung von 2-[(Alkoxycarbonyl)-amino]-1H-benzimidazolen der allgemeinen Formel V

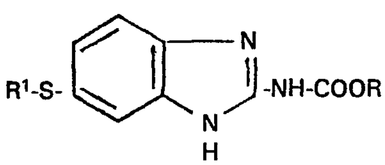

(V)

worin R und $R^1$ die oben angegebene Bedeutung haben, mit dem die oben angegebenen Schwierigkeiten in einfacher Weise umgangen werden. Das Verfahren der Erfindung ist dadurch gekennzeichnet, dass man einen (1H-Benzimidazol-2-yl)-carbaminsäurealkylester der allgemeinen Formel I

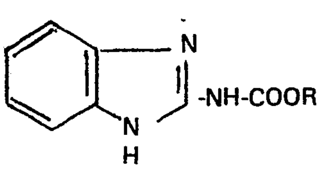

(I)

worin R die oben angegebene Bedeutung hat, in das 2-[(Alkoxycarbonyl)-amino]-1H-benzimidazol-5-sulfonsäurechlorid der allgemeinen Formel III

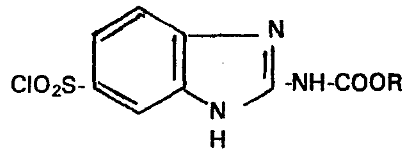

(III)

worin R die oben angegebene Bedeutung hat, überführt, indem man den (1H-Benzimidazol-2-yl)-carbaminsäurealkylester der Formel I entweder

a) mit Chlorsulfonsäure umsetzt oder

b) durch Umsetzen mit Oleum zunächst in die 2-[(Alkoxycarbonyl)-amino]-1H-benzimidazol-5-sulfonsäure der allgemeinen Formel II

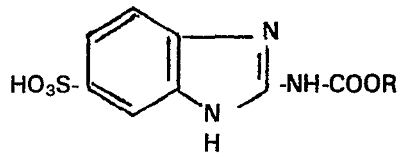

(II)

worin R die oben angegebene Bedeutung hat, überführt, die dann mit einem Chlorierungsmittel behandelt wird, das erhaltene 2-[(Alkoxycarbonyl)-amino]-1H-benzimidazol-5-sulfonsäurechlorid der Formel III zu dem 2-[(Alkoxycarbonyl)-amino]-1H-benzimidazol-5-thiol der allgemeinen Formel IV

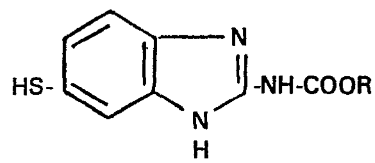

(IV)

worin R die oben angegebene Bedeutung hat, reduziert und in kristalliner Form isoliert und/oder durch Umsetzung mit einem Alkylhalogenid der Formel

$R^2$-Hal'

worin $R^2$ $C_{1-3}$-Alkyl und

Hal Halogen bedeuten,

in das 2-[(Alkoxycarbonyl)-amino]-5-(alkylthio)-1H-benzimidazol der allgemeinen Formel V überführt.

Einige Beispiele für Chlorierungsmittel für die Chlorierung der Benzimidazol-5-sulfonsäure der Formel II sind: Phosphoroxychlorid, Phosphorpentachlorid oder Brenzcatechylphosphortrichlorid.

Die Reduktion des Benzimidazol-5-sulfonsäurechlorids der Formel III führt man vorzugsweise in einem wässrigen, sauren Medium mit Metallen, wie z.B. Zinn, Zink und/oder Eisen, oder mit Metallsalzen, wie z.B. Stannochlorid, durch.

Die in dem Verfahren der Erfindung erhaltenen Verbindungen:

2-[(Alkoxycarbonyl)-amino]-1H-benzimidazol-5-sulfonsäurechlorid (Formel II) und dessen Salze,

2-[(Alkoxycarbonyl)-amino]-1H-benzimidazol-5-sulfonsäurechlorid (Formel II) und deren Salze,

2-[(Alkoxycarbonyl)-amino]-1H-benzimidazol-5-thiol (Formel IV) und dessen Salze sind neu.

Die Ümsetzung des (1H-Benzimdiazol-2-yl)-carbaminsäurealkylesters der Formel I gemäss Verfahrensvariante a) mit Chlorsulfonsäure wird nach einer zweckmässigen Ausführungsform der Erfindung im Temperaturbereich von etwa 15 bis etwa 40°C durchgeführt. Wird das so erhaltene Reaktionsgemisch in abs. Alkohol oder auf Eis gegossen, erhält man das 2-[(Alkoxycarbonyl)-amino]-1H-benzimidazol-5-sulfonsäurechlorid der Formel III in Form des Hydrochlorid-salzes. Wird das erhaltene Reaktionsgemisch in eine Mischung von Äther und Äthylacetat gegossen, erhält man das Benzimidazol-5-sulfonsäurechlorid der Formel III in Form des Schwefelsäuresalzes.

Die Umsetzung des (1H-Benzimidazol-2-yl)-carbaminsäure-alkylesters der Formel I mit Oleum gemäss Verfahrensvariante b) wird zweckmässig im Temperaturbereich von etwa 30 bis etwa 60°C durchgeführt und das erhaltene Reaktionsgemisch in Äther gegossen, wobei die 2-[(Alkoxycarbonyl)-amino]-1H-benzimidazol-5-sulfonsäure der Formel II in etwa 80%iger Ausbeute in Form von weissen Kristallen erhalten wird.

Zur Überführung der 2-[(Alkoxycarbonyl)-amino]-1H-benzimidazol-5-sulfonsäure der Formel II in das entsprechende Benzimidazol-5-sulfonsäurechlorid der Formel III gemäss Verfahrensvariante b) wird aus der Benzimidazol-5-sulfonsäure der Formel II zweckmässig zunächst mit Natriumhydroxyd in Methanol das Natriumsalz gebildet, das dann mit Phosphorpentachlorid bei etwa 110°C in eine Schmelze übergeführt und zur Isolierung auf Eis gegossen wird.

Die Reduktion des 2-[(Alkoxycarbonyl)-amino]-1H-benzimidazol-5-sulfonsäurechlorids der Formel III wird zweckmässig in saurem Medium mit Metallen, wie Zinn-, Zink- und/oder Eisenpulver, oder Metallsalzen, z.B. Stannochlorid, im Temperaturbereich von 0 bis etwa 100°C durchgeführt und das gebildete 2-[(Alkoxycarbonyl)-amino]-1H-benzimidazol-5-thiol der Formel IV in Form eines weissen, kristallinen Niederschlags isoliert.

Zur Umsetzung des Benzimidazol-5-thiols der Formel IV mit einem Alkylhalogenid wird das Benzimidazol-5-thiol der Formel IV zweckmässig in Dimethylformamid gelöst, mit einer wässrigen Alkalilösung bis zu pH 8 bis 9 versetzt, bei Raumtemperatur mit Alkylbromid umgesetzt, die erhaltene Lösung mit Wasser verdünnt und das ausgefallene 2-[(Alkoxycarbonyl)-amino]-5-alkylthio-1H-benzimidazol der Formel V isoliert.

Als wässrige Alkalilösung wird zweckmässig Natron- oder Kalilauge verwendet. Die Reaktion kann auch in Abwesenheit von Dimethylformamid in Wasser durchgeführt werden. Die Reaktionstemperatur liegt im Bereich von 40 - 45°C.

Das Verfahren der Erfindung weist gegenüber den vorbekannten Verfahren den Vorteil auf, dass als Ausgangsverbindungen der in grosstechnischem Massstab hergestellte und damit leicht erhältliche (1H-Benzimidazol-2-yl)-carbaminsäure-alkylester der Formel I verwendet werden kann, der durch technologisch einfach durchführbare Verfahren unter Verwendung von leicht und billig erhältlichen weiteren Verbindungen in das Benzimidazolderivat der Formel V übergeführt wird.

Weitere Einzelheiten des Verfahrens gemäss Erfindung sind den nachfolgenden Beispielen zu entnehmen.

*Beispiel 1*

*Herstellung von 2-[(Methoxycarbonyl)-amino]-1H-benzimidazol-5-sulfonsäure*

In einen 250 ml Vierhals-rundkolben, der mit einem Rührer, Thermometer und Schwefelsäure-blasenzähler ausgerüstet ist, gibt man 120 ml 20%iges Oleum und führt unter gelindem Kühlen mit Eiswasser innerhalb von 15 bis 20 Minuten bei 30 bis 40°C 38,4 g (0,2 Mol) (1H-Benzimidazol-2-yl)-carbaminsäure-methylester zu. Die Temperatur erhöht man auf 55 bis 60°C und rührt das Gemisch bei dieser Temperatur 2 Stunden. Inzwischen kühlt man 1.200 ml Äthyläther mit einer Eis-Kochsalzmischung auf —10°C und führt das Oleum-Reaktionsgemisch unter Rühren mit einer solchen Geschwindigkeit in den Äther ein, dass die Temperatur 0°C nicht übersteigt.

Das gewünschte Endprodukt erhält man in Form eines weissen, kristallinen Niederschlages, der abfiltriert, mit gekühltem Äther und mit 100 ml gekühltem abs. Äthanol gewaschen und getrocknet wird.

Gewicht: 43,5 (Th.: 54,26)
Ausbeute: 80%
Analyse:

| | | | | |
|---|---|---|---|---|
| ber.: | C 39,84 | H 3,34 | N 15,49 | S 11,8 |
| gef.: | C 39,2 | H 3,5 | N 15,1 | S 12,1 |

*Beispiel 2*

*Herstellung von 2-[(Methoxycarbonyl)-amino]-1H-benzimidazol-5-sulfonsäurechlorid.HCl*

In einen 500 ml Kolben, der mit einem Rührer ausgestattet ist, gibt man 81,3 g (0,3 Mol) 2-[(methoxycarbonyl)-amino]-1H-benzimidazol-5-sulfonsäure und 300 ml Methanol und tropft der Mischung eine 40%ige wässrige NaOH-Lösung bei Raumtemperatur bis zum pH von 7,5 zu, wobei die Ausgangsverbindung in Lösung geht und das Natriumsalz der 2-[(Methoxycarbonyl)-amino]-1H-benzimidazol-5-sulfonsäure ausfällt. Nach einer Stunde Rühren wird der Niederschlag abfiltriert, mit wenig Methanol gewaschen und getrocknet.

Gewicht: 90 g

Das trockene Natriumsalz der Sulfonsäure wird bei 110°C mit 130 g (0,625 Mol) Phosphorpentachlorid geschmolzen, weitere 2 Stunden bei dieser Temperatur gerührt, sodann auf 400 g Brucheis gegossen und zunächst mit Eiswasser, dann mit 100 ml abs. Äthanol von —10°C gewaschen und getrocknet.

Gewicht: 82 g (Th.: 97,86 g)
Ausbeute: 84%
Analyse:

| | | | | | |
|---|---|---|---|---|---|
| ber.: | C 33,14 | H 2,78 | Cl 21,74 | N 12,88 | S 9,83 |
| gef.: | C 32,9 | H 3,0 | Cl 21,6 | N 13,0 | S 9,75 |

*Beispiel 3*

*Herstellung von 2-[(Methoxycarbonyl)-amino]-1H-benzimidazol-5-sulfonsäurechlorid.HCl*

In einen 250 ml Vierhalskolben, der mit einem Rührer, Thermometer und Schwefelsäure-blasenzähler

ausgerüstet ist, gibt man 100 ml (178,7 g; 1,53 Mol) Chlorsulfonsäure, führt bei 15 bis 20°C innerhalb von 30 Minuten unter mildem Kühlen mit Eiswasser 38,4 g (0,2 Mol) (1H-Benzimidazol-2-yl)-carbaminsäuremethylester zu, erhitzt das erhaltene Gemisch auf 40°C und rührt es bei dieser Temperatur 2,5 Stunden. Das so erhaltene Reaktionsgemisch gibt man mit einer solchen Geschwindigkeit in 1.000 ml Äthanol von —10°C, dass die Temperatur nicht über 0°C steigt. Die Kühlung erfolgt mit einer Eis-Kochsalzmischung. Das gewünschte Endprodukt fällt in Form von weissen Kristallen aus, die filtriert, mit 100 ml gekühltem abs. Äthanol gewaschen und getrocknet werden.

Gewicht: 60,6 g (Th.: 65,2 g)
Ausbeute: 93%, Smp.: 250°C
Analyse:

| | | | | | |
|---|---|---|---|---|---|
| ber.: | C 33,14 | H 4,78 | Cl 21,74 | N 12,88 | S 9,83 |
| gef.: | C 32,94 | H 3,02 | Cl 21,64 | N 12,91 | S 9,81 |

*Beispiel 4*

*Herstellung von 2-[(Methoxycarbonyl)-amino]-1H-benzimidazol-5-sulfonsäurechlorid.HCl*

Es wird wie in Beispiel 3 verfahren, das gewünschte Endprodukt aber wie folgt isoliert:

Das Reaktionsgemisch der Chlorsulfonsäurebehandlung wird auf 800 g Brucheis gegossen, das ausgeschiedene Produkt abfiltriert, mit wenig Eiswasser und 100 ml auf —10°C gekühltem abs. Äthanol gewaschen und getrocknet.

Gewicht: 52,1 g (Th.: 65,2 g)
Ausbeute: 80%

Das aus dem Wasser isolierte Säurechlorid wird zweckmässig ohne zu trocknen weiter verarbeitet, da es sich während des Trocknens zersetzt.

*Beispiel 5*

*Herstellung von 2-[(Methoxycarbonyl)-amino]-1H-benzimidazol-5-sulfonsäurechlorid.$H_2SO_4$*

In einen 200 ml Vierhalskolben, der mit einem Rührer, Thermometer und Schwefelsäure-blasenzähler ausgestattet ist, werden 100 ml (178,7 g; 1,53 Mol) Chlorsulfonsäure gegeben, bei 15 bis 20°C innerhalb von etwa 30 Minuten unter Kühlen mit Eiswasser 38,4 g (0,2 Mol) (1H-Benzimidazol-2-yl)-carbaminsäuremethylester zugeführt und das erhaltene Gemisch auf 40°C erhitzt. Nach 2,5 Stunden Rühren bei dieser Temperatur führt man das Reaktionsgemisch in solcher Geschwindigkeit in eine auf —10°C gekühlte Lösung von 1.000 ml Äthyläther und 300 ml Äthylacetat ein, dass die Temperatur nicht über 0°C steigt.

Das ausgefallene weisse, kristalline Endprodukt wird abfiltriert, mit 200 ml gekühltem Äther gewaschen und in einem Vakuum-exsiccator getrocknet. (Das getrocknete Produkt ist nicht mehr hygroskopisch.)

Gewicht: 64,40 g (Th.: 77,4 g)
Ausbeute: 83,2%, Smp.: 142 bis 145°C (Zers.)
Analyse:

| | | | | | |
|---|---|---|---|---|---|
| ber.: | C 27,87 | H 2,6 | Cl 9,14 | N 10,83 | S 16,54 |
| gef.: | C 27,52 | H 2,8 | Cl 9,18 | N 10,12 | S 16,45 |

*Beispiel 6*

*Herstellung von 2-[(Methoxycarbonyl)-amino]-5-propylthio-1H-benzimidazol*

In 110 ml 40%ige Schwefelsäure gibt man bei 0°C 20 g Zinkpulver und dann unter Rühren 10,8 g (0,033 Mol) 2-[(Methoxycarbonyl)-amino]-1H-benzimidazol-5-sulfonsäurechlorid, rührt das erhaltene Gemisch zunächst 2 Stunden bei 0°C, erhitzt es dann auf 80 bis 90°C und rührt es danach eine weitere Stunde bei dieser Temperatur. man erhält 2-[(Methoxycarbonyl)-amino]-1H-benzimidazol-5-thiol in Form eines weissen, kristallinen Niederschlags, den man auf Raumtemperatur abkühlt, filtriert und mit wenig Eiswasser wäscht. Der Niederschlag wird in der fünffachen Menge Dimethylformamid gelöst, das nicht in Lösung gegangene Zink abfiltriert und mit Dimethylformamid gewaschen. Den pH-Wert der Lösung stellt man mit 40%iger Natronlauge auf 8 bis 9 ein, gibt 6 ml n-Propylbromid unter Kühlen mit Eiswasser zu, wobei man die Temperatur auf 25 bis 30°C hält. Das Gemisch wird 30 Minuten gerührt und dann 48 Stunden stehen gelassen. Danach wird die Lösung mit Wasser auf das fünffache Volumen verdünnt, der ausgefallene Niederschlag abfiltriert, mit Wasser und Aceton gewaschen und getrocknet, wobei man 5,50 g des gewünschten Endproduktes erhält.

Ausbeute: 62,8%

Nach Umkristallisieren aus Chloroform schmilzt das Produkt bei 208 bis 210°C (Zers.)

Analyse:

| | | | | |
|---|---|---|---|---|
| ber.: | C 54,32 | H 5,70 | N 15,84 | S 12,08 |
| gef.: | C 54,36 | H 5,64 | N 16,00 | S 11,92 |

*Beispiel 7*

*Herstellung von 2-[(Methoxycarbonyl)-amino]-5-propylthio-1H-benzimidazol*

10,8 g (0,033 Mol) 2-[(Methoxycarbonyl)-amino]-1H-benzimidazol-5-sulfonsäurechlorid.HCl werden in 35 ml Wasser suspendiert, mit 33 g $SnCl_2$ versetzt und 30 Minuten bei 50°C gerührt. Man erhält eine klare Lösung, aus der sich Kristalle abscheiden. Das Gemisch wird auf Raumtemperatur abgekühlt, der Niederschlag abfiltriert und mit wenig Wasser gewaschen. Das erhaltene 2-[(Methoxycarbonyl)-amino]-1H-benzimidazol-5-thiol wird nach Beispiel 6 weiter aufgearbeitet.

Ausbeute: 5 g, Smp.: 210°C.

*Beispiel 8*

*Herstellung von 2-[(Äthoxycarbonyl)-amino]-1H-benzimidazol-5-sulfonsäurechlorid.HCl*

Zu 100 ml (178,8 g; 1,53 Mol) Chlorsulfonsäure gibt man bei 15 bis 20°C 41 g (0,2 Mol) (1H-Benzimidazol-2-yl)-carbaminsäureäthylester innerhalb von etwa 30 Minuten, erhitzt das Gemisch auf 40°C und rührt es 2,5 Stunden. Das so erhaltene Reaktionsgemisch giesst man in abs. Äthanol von —10°C, wobei man das gewünschte Endprodukt in Form von weissen Kristallen erhält, die abfiltriert, mit Äthanol gewaschen und getrocknet werden.

Gewicht: 59 g (Th.: 68 g)

Analyse:

ber.: C 35,3 H 3,26 Cl 20,84 N 12,35 S 9,43

gef.: C 35,0 H 3,1 Cl 20,6 N 12,7 S 9,3

*Beispiel 9*

*Herstellung von 2-[(Äthoxycarbonyl)-amino]-5-äthylthio-1H-benzimidazol*

In 110 ml 40%ige Schwefelsäure führt man bei 0°C 20 g Zinkpulver unter Rühren ein, gibt dann 11,2 g (0,033 Mol) 2-[(Äthoxycarbonyl)-amino]-1H-benzimidazol-5-sulfonsäurechlorid.HCl zu und rührt das Gemisch 2 Stunden bei 0°C, erhitzt es auf 80-90°C und rührt es danach eine weitere Stunde bei dieser Temperatur, wonach 2-[(Äthoxycarbonyl)-amino]-1H-benzimidazol-5-thiol in Form von weissen Kristallen ausfällt. Der kristalline Niederschlag wird in der fünffachen Menge Dimethylformamid gelöst, das ungelöste Zinkpulver abfiltriert und der pH-Wert der Lösung mit wässriger Natronlauge auf 8 bis 9 eingestellt, wonach man 5 ml Äthylbromid zugibt und das Gemisch 48 Stunden stehen lässt. Nach Verdünnen mit Wasser fällt das gewünschte Endprodukt aus, das abfiltriert, mit Wasser und Aceton gewaschen wird.

Gewicht: 4,4 g

Analyse:

ber.: C 54,32 H 5,70 N 15,84 S 12,08

gef.: C 54,2 H 5,52 N 15,90 S 11,85

*Beispiel 10*

*Herstellung von 2-[(Methoxycarbonyl)-amino]-5-propylthio-1H-benzimidazol*

In 100 ml 10%ige Salzsäure gibt man im Temperaturbereich von 0 bis 10°C zunächst 1,5 g Zinnpulver, sodann 10,8 g (0,033 Mol) 2-[(Methoxycarbonyl)-amino]-1H-benzimidazol-5-sulfonsäurechlorid. HCl zu und rührt das erhaltene Gemisch bei dieser Temperatur eine Stunde. Danach gibt man 10 g Eisenpulver zu und rührt weitere 6 Stunden bei 25 bis 30°C. Die Metalle lösen sich auf und 2-[(Methoxycarbonyl)-amino]-1H-benzimidazol-5-thiol scheidet sich ab, das filtriert, in 10%iger Kaliumhydroxydlösung gelöst und bei 40 bis 45°C mit 6 ml n-Propylbromid umgesetzt wird. Das ausgefallene Endprodukt wird abfiltriert, mit Wasser und Aceton gewaschen und getrocknet.

Gewicht: 4 g, Smp.: 213 bis 214°C (Zers.)

*Beispiel 11*

*Herstellung von 2-[(Methoxycarbonyl)-amino]-5-propylthio-1H-benzimidazol*

In 100 ml 10%ige Salzsäure gibt man bei Raumtemperatur 10,8 g (0,033 Mol) 2-[(Methoxycarbonyl)-amino]-1H-benzimidazol-5-sulfonsäurechlorid. HCl und 10 g Eisenpulver, rührt das erhaltene Gemisch 5 Stunden bei 70 bis 80°C, filtriert das ausgefallene 2-[(Methoxycarbonyl)-amino]-1H-benzimidazol-5-thiol ab und führt es in der in Beispiel 10 angegebenen Weise in 2-[(Methoxycarbonyl)-amino]-5-propylthio-1H-benzimidazol über.

Smp. 208 bis 210°C.

*Beispiel 12*

*Herstellung von 2-[(Methoxycarbonyl)-amino]-1H-benzimidazol-5-thiol*

In 100 ml 10%ige Salzsäure gibt man bei 0 bis 10°C 6 g Zinnpulver und führt 10,8 g (0,033 Mol) 2-[(Methoxycarbonyl)-amino]-1H-benzimidazol-5-sulfonsäurechlorid.HCl zu, rührt das Gemisch 2 Stunden unterhalb von 10°C und 10 Stunden bei 25°C. Die Ausgangsverbindungen lösen sich dabei auf und das gewünschte Endprodukt fällt aus.

Gewicht: 6 g (81,5%), Smp.: 214 bis 215°C (Zers.)

Analyse:

ber.: C 48,60 H 4,06 N 18,88 S 14,39

gef.: C 48,22 H 4,20 N 18,52 S 13,80

**Patentansprüche**

1. Verfahren zur Herstellung von Benzimidazolderivaten der allgemeinen Formel V

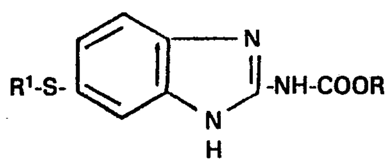 (V)

worin

R $C_1$-$C_3$-Alkyl und

$R^1$ $C_1$-$C_3$-Alkyl oder Wasserstoff bedeuten,

dadurch gekennzeichnet, dass man einen Benzimidazol-carbaminsäure-alkylester der allgemeinen Formel I

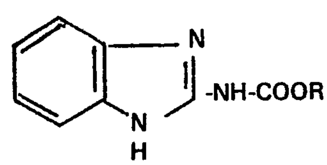 (I)

worin

R die oben angegebene Bedeutung hat,

in das Benzimidazol-5-sulfonsäurechlorid der allgemeinen Formel III

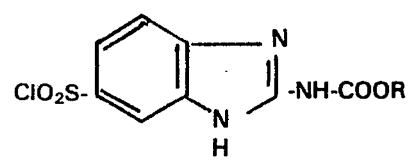 (III)

worin

R die oben angegebene Bedeutung hat,

überführt, indem man den Benzimidazol-carbaminsäure-alkylester I entweder

a) mit Chlorsulfonsäure umsetzt oder

b) durch Umsetzen mit Oleum zunächst in die Benzimidazol-5-sulfonsäure der allgemeinen Formel II

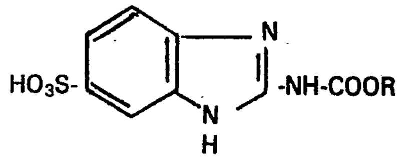 (II)

worin
R die oben angegebene Bedeutung hat,
überführt, die dann mit einem Chlorierungsmittel behandelt wird, das erhaltene Benzimidazol-5-sulfonsäurechlorid III zu dem Benzimidazol-5-thiol der allgemeinen Formel IV

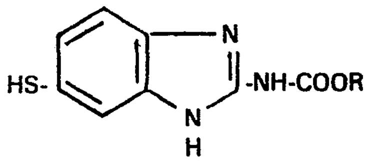
(IV)

worin
R die oben angegebene Bedeutung hat,
reduziert und in kristalliner Form isoliert und/oder mit einem Alkylhalogenid der Formel

$R^2Hal$

worin
$R^2$ $C_1$-$C_3$-Alkyl und
Hal Halogen bedeuten,
zu dem Benzimidazolderivat der allgemeinen Formel V umsetzt.

2. Verfahren nach Anspruch 1, dadurch gekennzeichnet, dass man für die Chlorierung der Benzimidazol-5-sulfonsäure II als Chlorierungsmittel Phosphoroxychlorid, Phosphorpentachlorid oder Benzcatechylphosphortrichlorid verwendet.

3. Verfahren nach Anspruch 1, dadurch gekennzeichnet, dass man die Reduktion des Benzimidazol-5-sulfonsäurechlorids II in wässrigem, saurem Medium mit Metallen oder Metallsalzen durchführt.

**Claims**

1. Process for the preparation of benzimidazole derivatives of general formula V

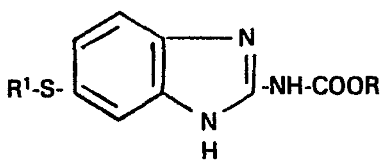
(V)

wherein
R represents $C_{1-3}$ alkyl and
$R^1$ represents $C_{1-3}$ alkyl or hydrogen,
characterized in that an alkyl benzimidazole carbaminate of general formula I

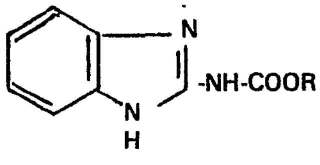
(I)

wherein
R is as hereinbefore defined,
is converted into the benzimidazole-5-sulphonic acid chloride of general formula III

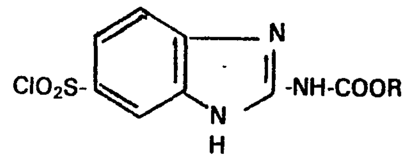
(III)

wherein
R is as hereinbefore defined,
by either
a) reacting the alkyl benzimidazole carbaminate of formula I with chlorosulphonic acid or
b) converting it (by reaction with oleum) first into the benzimidazole-5-sulphonic acid of general formula II

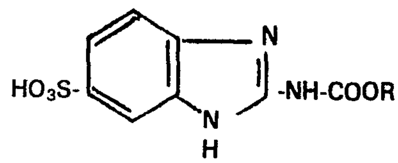
(II)

wherein
R is as hereinbefore defined,
which is then treated with a chlorinating agent, reducing the benzimidazole-5-sulphonic acid chloride of general formula III obtained in order to obtain the benzimidazole-5-thiol of general formulaIV

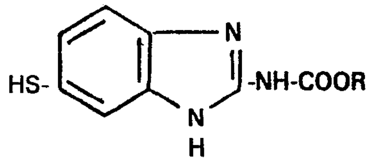
(IV)

wherein
R is as hereinbefore defined,
and isolating said product in crystalline form, and/or reacting it with an alkylhalide of formula

$R^2Hal$

wherein
$R^2$ represents $C_{1-3}$ alkyl and
Hal represents halogen,
to obtain the benzimidazole derivative of general formula V.

2. Process as claimed in claim 1, characterized in that phosphorus oxychloride, phosphorus pentachloride or pyrocatechyl phosphorus trichloride is used as chlorinating agent for the chlorination of the benzimidazole-5-sulphonic acid of formula II.

3. Process as claimed in claim 1, characterized in that the reduction of the benzimidazole-5-sulphonic acid chloride of formula II is effected in an aqueous acidic medium with metal or metal salts.

**Revendications**

1. Procédé de préparation de dérivés de benzimidazole de formule générale V

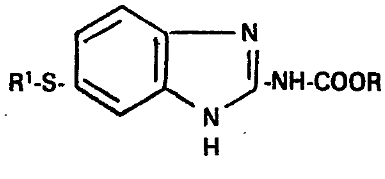
(V)

où

R représente un alcoyle en $C_1$ à $C_3$ et

$R^1$ représente un acoyle en $C_1$ à $C_3$ ou un hydro gène,

caractérisé en ce qu'on transforme un alcoylester d'acide benzimidazole-carbamique de formule générale I

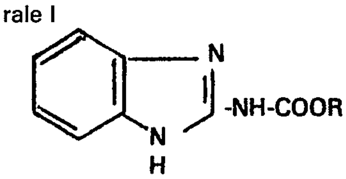

(I)

où

R a la signification donnée ci-dessus,

en le chlorure de l'acide benzimidazole-5-sulfonique de formule générale III

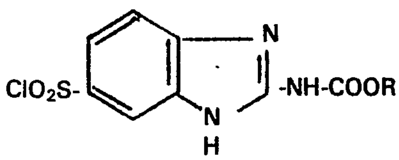

(III)

où

R a la signification donnée ci-dessus

en faisant réagir l'alcoylester de l'acide benzimidazole-carbamique I soit

a) avec de l'acide chlorosulfonique, soit

b) par réaction avec de l'oléum pour donner tout d'abord l'acide benzimidazole-5-sulfonique de formule générale II

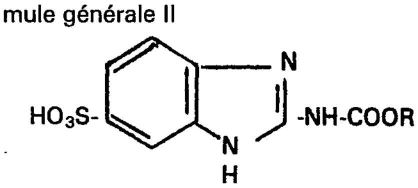

(II)

où

R a la signification donnée ci-dessus,

que l'on traite alors avec un agent chlorant, en réduisant le chlorure de l'acide benzimidazole-5-sulfonique III obtenu pour donner le benzimidazole-5-thiol de formule générale IV

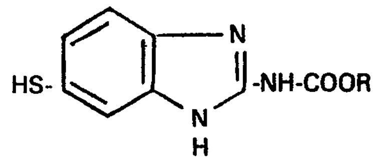

(IV)

où

R a la signification donnée ci-dessus,

et en l'isolant sous forme cristalline et/ou en le transformant par réaction avec un halogénure d'alcoyle de formule

$R^2Hal$

où

$R^2$ représente un alcoyle en $C_1$ à $C_3$ et

Hal représente un halogène,

pour donner le dérivé de benzimidazole de formule générale V.

2. Procédé selon la revendication 1, caractérisé en ce que pour la chloruration de l'acide benzimidazole-5-sulfonique II on utilise comme agent chlorant l'oxychlorure de phosphore, le pentachlorure de phosphore ou le trichlorure de pyrocatéchylphosphore.

3. Procédé selon la revendication 1, caractérisé en ce qu'on conduit la réaction du chlorure de l'acide benzimidazole-5-sulfonique II en milieu acide aqueux avec des métaux ou des sels métalliques.